Europäisches Patentamt

European Patent Office

Office européen des brevets

(11) Numéro de publication: **0 274 939**
**A2**

## (12) DEMANDE DE BREVET EUROPEEN

(21) Numéro de dépôt: 87402811.1

(22) Date de dépôt: **11.12.87**

(51) Int. Cl.4: **C07K 1/14 , C07K 1/12 ,
A61K 37/18 , A23L 1/305 ,
B01D 15/08**

(30) Priorité: **15.12.86 FR 8617515**

(43) Date de publication de la demande:
**20.07.88 Bulletin 88/29**

(84) Etats contractants désignés:
**AT BE CH DE ES FR GB GR IT LI LU NL SE**

(71) Demandeur: **Société anonyme dite:
LABORATOIRE ROGER BELLON
159, avenue A. Peretti
F-92201 Neuilly-sur-Seine(FR)**

(72) Inventeur: **Chataud, Jean
Route de Chinon Noyant de Touraine
F-37800 Sainte Maure de Touraine(FR)**
Inventeur: **Desreumaux, Serge
La Cholleterie
F-37250 Veigne(FR)**
Inventeur: **Cartwright, Terence
Le Moulin de l'Etang
F-37250 Saint Epain(FR)**

(74) Mandataire: **Portal, Gérard et al
Cabinet Beau de Loménie 55, rue
d'Amsterdam
F-75008 Paris(FR)**

(54) **Procédé de préparation d'un mélange peptidique riche en di- et tripeptides utilisable notamment en nutrition artificielle et en diététique, mélange ainsi obtenu, et utilisation de ce mélange en nutrition artificielle et en diététique.**

(57) 1. Procédé de préparation d'un mélange peptidique riche en di-et tri-peptides, à partir d'un hydrolysat enzymatique de protéines ; un tel procédé comprend :
- la chromatographie de cet hydrolysat sur une colonne d'une résine échangeuse de cations sous forme acide,
- le lavage de la colonne de résine échangeuse par de l'eau,
- l'élution de ladite colonne de résine échangeuse par une solution aqueuse d'un alcali,
- et la récupération de la fraction de l'éluat enrichie en di-et tri-peptides, en particulier celle qui sort de la colonne de résine échangeuse depuis le moment où l'indice de réfraction de cet éluat a atteint la valeur 5 jusqu'au moment où le pH de cet éluat a atteint la valeur 8.
Le mélange peptidique riche en di-et tri-peptides selon l'invention est utilisable avantageusement en nutrition artificielle et en diététique.

Fig. 2

1a

## Procédé de préparation d'un mélange peptidique riche en di-et tri-peptides, utilisable notamment en nutrition artificielle et en diététique, mélange ainsi obtenu, et utilisation de ce mélange en nutrition artificielle et en diététique.

La présente invention concerne un procédé de fabrication des mélanges riches en di-et tri-peptides utilisables notamment en nutrition artificielle orale, entérale ou parentérale, et en diététique, à partir d'hydrolsats enzymatiques de protéines.

Il est de nos jours admis et démontré que les di-et tri-peptides présentent un réel intérêt en nutrition artificielle que celle-ci soit de nature orale ou entérale. D'autres expériences semblent montrer qu'il y aurait un intérêt également en nutrition parentérale.

La nutrition artificielle est une technique appliquée aux patients incapables des s'alimenter par la voie normale en raison de dommages physiques (accident, opération chirurgicale, cancer de l'oesophage, etc) ou en raison d'un état physiologique générale qui ne permet pas une alimentation normale (coma, infection sévère, trauma, brûlures, etc).

La nutrition artificielle peut être donnée par la voie naturelle (voie digestive), il s'agit alors de nutrition orale ou entérale. Dans d'autres cas, les nutriments sont introduits directement dans la circulation sanguine, il s'agit alors de nutrition parentérale. Ces différents modes d'administration (orale, entérale, parentérale) sont réalisés selon des protocoles et des techniniques bien établis, connus à l'hôpital, et nécessitent un matériel adéquat (sondes, pompes, cathéter), le choix du type d'alimentation dépend de l'état du malade ; dans certains cas, sa mise en oeuvre doit faire appel à une équipe médicale spécialisée (réanimation). Plus rarement, la nutrition artificielle est faite à domicile.

Une nutrition complète nécessite l'administration de l'azote sous la forme de protides (protéines, peptides, aminoacides) en mélange avec des lipides, des hydrates de carbone, des éléments minéraux et des vitamines.

Dans une alimentation normale, le majeure partie de l'azote consommé est ingérée sous la forme de protéines et est digérée par les enzymes du tractus digestif.

Toutefois, dans un grand nombre de situations citées où la nutrition artificielle est utilisée, la digestion effective des protéines n'est pas possible du fait de déficiences enzymatiques dues aux maladies ou à la portion limitée de l'intestin restant fonctionnelle après la maladie ou l'intervention chirurgicale. Ce problème a été résolu de manière classique par l'emploi de solutions d'aminoacides purs.

En nutrition artificielle, ces mélanges d'aminoacides présentent toutefois de nombreux inconvénients théoriques et pratiques :
- les aminoacides sont transportés depuis l'intestin par des processus de transport actif. Différents aminoacides entrent en compétition pour le transport, et l'absoprtion intestinale peut être limitée par cette compétition ;
- certains états pathologiques peuvent perturber le transport de certains aminoacides, provoquant une réduction de la capacité d'absorption ;
- certains aminoacides, notamment la tyrosine et la cystine, sont peu solubles dans l'eau et ce manque de solubilité limite la disponibilité de ces matières nutritives importantes ;
- certains aminoacides existent sous forme d'amides dans les protéines (asparagine et glutamine). Ces aminoacides amidés sont instables en solution et se désaminent en acide aspartique et en acide glutamique respectivement ; la glutamine, en particulier, bien qu'importante en nutrition artificielle est ainsi déficiente dans les mélanges classiques d'aminoacides ;
- pour l'utilisation intraveineuse, une solution d'aminoacides suffisamment concentrée pour transporter la quantité d'azote requise a une osmolarité qui est trop élevée pour l'injection par les veines superficielles usuelles car elle provoque des dommages aux tissus (les phlébites en sont l'exemple le plus courant).

Ce problème peut être évité par l'implantation de cathéters dans les veines profondes.

Toutefois, ce procédé chirurgical est compliqué, et présente un risque septique important. Ces problèmes peuvent limiter sérieusement l'utilisation intraveineuse des solutions nutritives à base d'aminoacides.

Il a été montré récemment (Silk D.B.A. Peptide absorption in man-Gut 15 194 (1974) ; Matthews D.M. Intestinal absorption of peptides - Phys. Rev. 55 537 (1975) ; Adibi S.A. et Y.S. Kim Peptide absorption and hydrolysis in Physiology of the gastrointestinal tract. Ed. L.R. Johnson, Raven Press New York 1073) que la molécule de peptide comprenant de 2 à 3 aminoacides liés par des liaisons peptidiques (di-ou tri-peptide respectivement) est absorbée à l'état intact à partir de l'intestin par un mécanisme distinct de celui mis en oeuvre dans le transport des aminoacides.

Il apparaît que les cellules de surface dans l'intestin absorbent les di-et tri-peptides, les hydrolysent in situ en aminoacides et libèrent les aminoacides ainsi obtenus dans le sang. Il existe, au niveau rénal, un processus similaire (réabsorption avec hydrolyses des di-et tri-peptides et passage des aminoacides dans la circulation sanguine).

Compte tenu de ce qui précède, un mélange nutritif qui contient des di-et tri-peptides comme source d'azote présente plusieurs avantages liés à :
- la stabilité des aminoacides amidés,
- la forte teneur en azote due à la bonne solubilité de di-et tri-peptides,
- l'osmolarité faible.

La digestion enzymatique des protéines a été largement utilisée comme un moyen de production de mélanges de peptides inférieurs ou "petits peptides".

Ainsi toutes les préparations décrites contiennent un large spectre de longueurs de chaîne peptidique (de 2 à plus de 20 aminoacides) mais la teneur en di-et tri-peptides spécialement requise est très fiable (de l'ordre de 5 à 20 %).

Des peptides supérieurs aux di-et tri-peptides ne montrent pas les avantages de l'absorption cinétique décrite précédemment et ainsi ces mélanges de peptides mal définis ne produisent pas une absorption d'azote optimale dans les utilisations cliniques.

Pour l'utilisation intraveineuse, les mélanges contenant des quantités appréciables de peptides supérieurs ne sont pas satisfaisants pour deux raisons supplémentaires :
- en premier lieu, les peptides supérieurs à 3 aminoacides sont excrétés dans l'urine, sans réabsorption subséquente. L'utilisation d'azote est ainsi très inefficace avec des pertes pouvant atteindre 60%,
- en second lieu, les peptides supérieurs comportent le risque de présenter des sites antigéniques intacts avec la possibilité de provoquer la production d'anti-corps ou même une réaction anaphylactique.

Ainsi, les hydrolysats enzymatiques de protéines classiques ne conviennent pas pour la production de mélanges contenant seulement des di-et tri-peptides.

On peut également préparer les di-et tri-peptides par synthèse chimique et l'expérience a montré que ces mélanges de di-peptides peuvent être utilisés d'une manière efficace dans la nutrition artificielle (FURST P. Peptides in parenteral nutrition - Clinical Nutrition 4 (Suppl. 1985) p. 105 ; KRZYSIK B.A. et ADIBI S.A. Comparison of metabolism of glycine injected intravenously in free and dipeptide forms ; STEINHARDT H.J., PALEOS G.A., BRANDL M. , FEKL W.L. , ADIBI S.A. - Efficacy of synthetic dipeptide mixture as the source of amino acids for total parenteral nutrition in a subhuman primate).

Toutefois, cette approche présente également des inconvénients :
- en premier lieu, la totalité des di-peptides possibles (au nombre de 400) ne peut pas être raisonnablement synthétisée; l'expérimentation était donc basée sur un nombre limité de di-peptides contenant la glycine (c'est-à-dire glycine - X, dans laquelle X est un autre amino acide).

Cette approche limite les di-peptides disponibles et peut entraîner un déséquilibre dû à l'excès de glycine.
- en second lieu, une autre limite de l'approche par synthèse chimique est le coût de production des di-peptides qui est trop élevé pour un usage en nutrition.

Le présent invention permet de remédier aux inconvénients des procédés classiques exposés ci-dessus, et concerne un procédé pour la production des mélanges peptidiques contenant principalement des di-et tri-peptides à partir d'un hydrolysat enzymatique de protéines par l'extraction chromatographique des di-et tri-peptides sous une forme concentrée non pyrogène, pouvant convenir pour l'usage clinique en nutrition artificielle et avec une teneur en éléments minéraux compatible avec l'administration par voie intraveineuse. Les mélanges peptidiques ainsi obtenus peuvent aussi être utilisés en alimentation orale et entérale.

Comme déjà mentionné ci-dessus, en alimentation orale ou entérale les di-et tri-peptides offrent plusieurs avantages par rapport aux nutriments ordinaires (aussi dénommés polymériques) et aux diètes élémentaires (qui con tiennent les protides sous forme d'aminoacides).

Le premier avantage est d'ordre cinétique ; au niveau intestinal, l'apport azoté est plus rapide lorsque les protides sont sous forme de di-et tri-peptides que dans le cas où ceux-ci sont sous forme élémentaire ou ologomérique (produits de la digestion gastrique et pancréatique).

Un deuxième avantage tient à la non compétition au niveau de l'absorption qui existe entre les di-et tri-peptides d'une part et les aminoacides d'autre part.

Un troisième avantage tient à la très bonne solubilité des petits peptides (haute teneur en tyrosine ; possibilité d'avoir des solutés à plus de 25 g d'azote par litre).

Un quatrième avantage est lié au faible rapport osmolarité/azote (deux à trois meilleur à celui des solutés d' aminoacides).

On peut donc concevoir que dans certaines conditions pathologiques où les systèmes de transport des aminoacides sont perturbés. l'absorption des di-et tri-peptides peut ne pas être modifiée.

L'intérêt des di-et tri-peptides en alimentation parentérale tient en trois points :

1°. Un soluté de di-et tri-peptides en quantité d'azote égale à celle d'un soluté d'aminoacides présente une osmolarité deux à trois fois plus faible.

Ceci est particulièrement important car il permet un apport azoté suffisant aux besoins nutritifs des malades par le voies périphériques, ce qui est impossible avec les solutés d'aminoacides.

De plus, la faible osmolarité des solutés de di-et tri-peptides diminue nettement les risques de complication de type phlébite et autres pathologies du système circulatoire.

Enfin, et surtout. l'utilisation des vaisseaux périphériques à la place des vaisseaux profonds est plus aisée et beaucoup moins dangereuse en ce qui concerne le risque septique.

2°. Les solutés d'aminoacides ne contiennent pas de glutamine car cet aminoacide primordial en matière d'anabolisme est instable dans l'eau. Par contre, les peptides qui contiennent de la glutamine sont beaucoup plus stables en solution aqueuse.

Ce qui est dit de la glutamine est également valable pour l'asparagine.

3°. Certains aminoacides sont en quantité limitée dans les solutés d'aminoacides pour des raisons de solubilité ; le cas le plus caractéristique est celui de la tyrosine.

Les di-et tri-peptides contenant de la tyrosine ont une solubilité qui permet de subvenir aux besoins en tyrosine des malades relevant d'une alimentation parentérale.

Ce qui est dit de la tyrosine est valable pour d'autres aminoacides dont la solubilité est également faible.

La présente invention a pour objet un procédé de préparation d'un mélange peptidique riche en di-et tri-peptides à partir d'un hydrolysat enzymatique de protéines contenant des di-, tri-et oligopeptides supérieurs aux tripeptides, ledit procédé comprenant :

- la chromatographie de cet hydrolysat sur une colonne d'une résine échangeuse de cations sous forme acide,
- le lavage de la colonne de résine par de l'eau. de préférence stérile, apyrogène,
- l'élution de ladite colonne de résine échangeuse par une solution aqueuse d'un alcali,
- et la récupération de la fraction de l'éluat enrichie en di-et tripeptides.

Plus particulièrement. la fraction de l'éluat enrichie en di-et tri-peptides est celle qui sort de la colonne de résine échangeuse depuis le moment où l'indice de réfraction de cet éluat a atteint la valeur 5, et jusqu'au moment où le pH de cet éluat a atteint la valeur 8, moment auquel la récupération de ladite fraction est terminée.

L'hyrolysat enzymatique de protéines qui est la matière de départ. utilisée dans le procédé selon la présente invention est un produit de digestion enzymatique des protéines qui a été ultrafiltré et qui contient à la fois des peptides et des aminoacides. lesdits peptides incluant les dipeptides, les tripeptides et les oligopeptides supérieurs aux tripeptides.

D'un point de vue industriel. les protéines utilisables pour la fabrication de cet hydrolysat comprennent à titre d'exemple les suivantes :
- blanc d'oeuf (ovalbumine)
- protéines laitières : lactalbumines et caséines
- protéines d'abattoirs : sérum albumine, hémoglobine décolorée
- produits de l'industrie des pêches et de la conserverie de poissons
- des produits d'origine végétale : protéines de soja et de luzerne.

Du point de vue industriel, les enzymes utilisables pour cette fabrication, à titre d'exemple, peuvent être les suivantes :
- enzymes d'origine micro-organique que l'on a coutume de subdiviser en trois catégories :
. celles utilisables en milieu acide (pH 1,5-4) ;
. celles utilisables à un pH voisin de la neutralité (pH 5,0 - 8,0) ;
. celles utilisables en milieu alcalin (pH 7-11).

La Molsin® Fujisawa C.K.K. et la Sanprose® Hankyu Kyoei Bussan K.K. appartiennent à la première catégorie : la Neutrase® Novo, la Protéase B500® Rapidase Gist Brocades et la H.T. Protéolytique® Miles appartiennent à la seconde catégorie ; l'Alcalase® Novo, la Protéase A2® Rapidase Gist Brocades et l'Optimase® Miles appartiennent à la troisième catégorie.
- enzymes d'origine animale : essentiellement la pepsine, la chymotrypsine, la trypsine
- enzymes d'origine végétale : les plus utilisées sont la papaïne, la ficine, la broméline.

L'action de l'une ou de plusieurs de ces enzymes sur l'une ou plusieurs de ces protéines conduit à des hydrolysats pouvant contenir de 10 a 50 % de di-et tri-peptides.

Les résines échangeuses de cations sous forme acide utilisables selon la présente invention doivent répondre aux critères suivants :

4

- correspondre aux besoins de décationisation (dessalage) et de séparation peptidique
- présenter une excellente résistance physique et chimique
- présenter une grande stabilité et une longue durée d'utilisation
- avoir une capacité d'échange élevée
- avoir des conditions d'utilisation de pH et température larges
- avoir une capacité de gonflement minimum
- être de régénération facile
- être d'un prix de revient acceptable
- être d'approvisionnement facile
- être de qualité reproductible.

On peut utiliser les résines échangeuses de cations sous forme acide, de type classique, utilisées en déminéralisation classique ou en séparation des acides aminés ; en particulier, on peut utiliser les résines échangeuses de cations, fortement acides, à squelette polystyrénique, portant des groupements acides sulfoniques, présentées sous forme de solides macroporeux, et notamment celles commercialisées sous les marques suivantes :

"Duolite" de Duolite International

"Amberlite" de Rohm et Haas,

"Dowex" de Dow Chemical.

"Lowatit" de Bayer.

Les résines cationiques fortement acides, à structure macroporeuse, suivantes sont préférées :
- Duolite ® type XE 521-251 ( copolymère styrène-divinylbenzène à groupements acides sulfoniques ; densité apparente 0,85 g/cm$^3$ ; granulométrie comprise entre 0,2 et 0,7 mm).
- Amberlite ® type 200C (copolymère styrène-divinylbenzène à groupements acides sulfoniques ; densité apparente 0,80 g/cm3 ; granulométrie 0,45-0,60 mm).

La solution aqueuse d'alcali utilisée pour l'élution de la colonne de résine échangeuse peut être une solution de soude ou de potasse ayant une concentration égale ou inférieure à 2N.

La quantité d'hydrolysat à charger sur la colonne de résine échangeuse est égale ou inférieure à celle nécessaire pour saturer complètement ladite résine échangeuse ; la saturation de cette résine échangeuse est obtenue avec 400 g environ de protides par litre de résine échangeuse.

A titre d'exemple, les conditions optimales à observer sont les suivantes :
- volume de la résine échangeuse : V
- température : entre 4 et 80°C et de préférence entre 60 et 80°C
- débit : de 1,5 à 3 V·h
- chargement de l'hydrolysat : 2 à 5 V (selon la concentration en protides de l'hydrolysat, correspondant à 400 g de protides par litre de résine échangeuse)
- lavage H$_2$O : 2 à 3 V
- élution :
- concentration de la solution d'alcali : KOH : de 0,5 à 2 N et de préférence N
ou NaOH : de 0,5 à 2 N et de préférence N
- récupération de la fraction d'éluat riche en di-et tri-peptides :
début : IR = 5
fin : pH - 8

La fraction de l'éluat enrichie en di-et tri-peptides peut éventuellement être purifiée par ultra-filtration, concentrée ou diluée si nécessaire, puis par filtration stérilisante et conditionnée sous forme d'un liquide, ou bien elle peut être séchée sous la forme d'une poudre.

Cette fraction de l'éluat enrichie en di-et tri-peptides, sous forme purifiée, liquide ou en poudre constitue le mélange peptidique riche en di-et tri-peptides selon la présente invention. Ce mélange est utilisable en nutrition artificielle et en diététique, et plus particulièrement en nutrition parentérale.

Les principales caractéristiques de ce produit sont :
- liquide stérile et apyrogène
- teneur en azote jusqu'a 25 grammes/litre
- osmolarité inférieure à 400 milliosmoles par litre à 12 g/l d'azote
- teneur en sodium, potassium, calcium pratiquement nulle
- teneur en chlorures inférieure à 15 meq/l
- teneur en ammoniaque inférieure à 5 meq/l pour un produit non autoclavé (filtration stérilisante) et de l'ordre de 10 meq/litres pour un produit autoclavé
-teneur en di-, tri-, tétrapeptides de l'ordre de 90 % en poids par poids
- teneur en di-, tri-peptides supérieure à 70 % en poids par poids

- teneur en aminoacides libres égale ou inférieure à 5 %.

L'originalité de l'invention tient au fait qu'en un seul traitement l'hydrolysat enzymatique de protéines est désionisé et fractionné de sorte que l'on obtient une fraction très enrichie en di-et tri-peptides.

La présente invention concerne également les mélanges peptidiques riches en di-et tri-peptides obtenus directement par le procédé selon l'invention.

On décrit l'invention en référence au dessin ci-joint sur lequel :

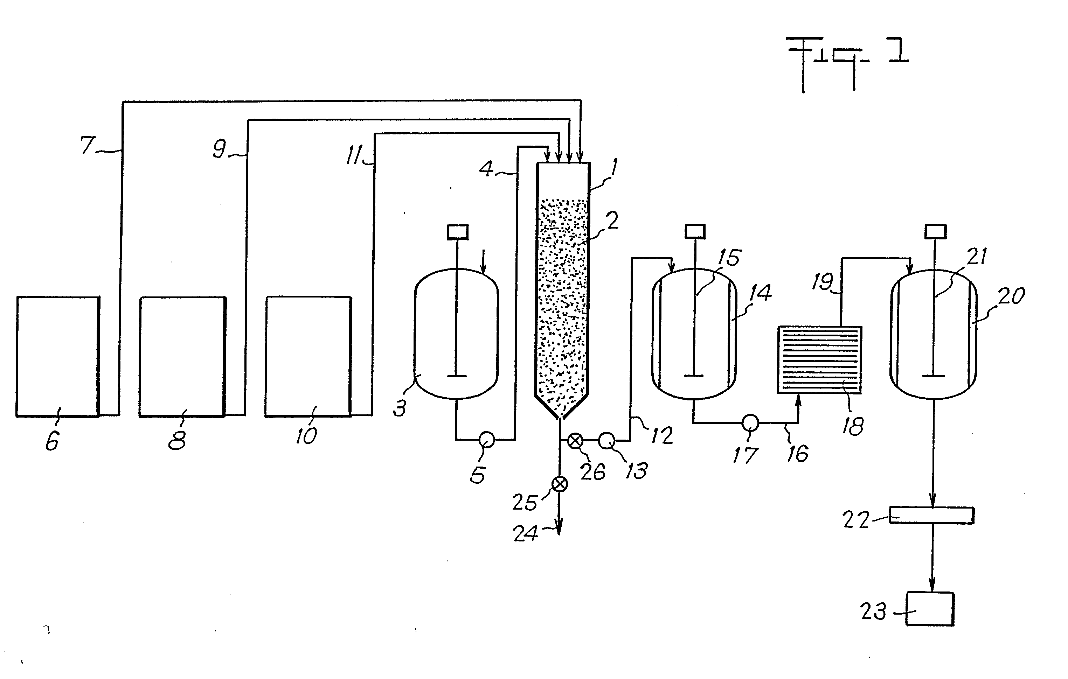

la figure 1 représente un schéma de l'installation pour la mise en oeuvre du procédé selon l'invention.

la figure 2 est un graphique pour suivre l'évolution de l'indice de réfraction IR et du pH de l'effluent de la colonne de résine échangeuse.

Selon ce graphique, la séparation-purification s'effectue en quatre étapes. L'étape A correspond au chargement de la colonne en hydrolysat de protéine ultrafiltré ; l'étape B à un lavage à l'eau avec sortie des peptides non retenus. Les étapes C et D sont celles de la chromatographie proprement dite (déplacement par une solution alcaline des peptides retenus). L'effluent sortant dans les étapes a et B contient, outre les anions, les peptides les plus gros de l'hydrolysat ultrafiltré (premier pic). La taille moyenne de ces peptides est comprise entre 10 et 15 résidus. L'effluent sortant à l'étape C (début second pic) contient, sous forme diluée des peptides intermédiaires (octa, hepta, hexa, penta). Au-delà de l'étape D sortent en mélange avec les petits peptides les cations de l'hydrolysat (Na , K , NH₄ ).

L'installation pour la mise en oeuvre du procédé selon l'invention comporte une colonne verticale 1, remplie d'un chargement 2 d'une résine échangeuse de cations sous forme acide. Cette colonne est alimentée à sa partie supérieure, et séparément :

- d'une part, en hydrolysat de protéine ultrafiltré provenant de la cuve 3 via la conduite 4 et la pompe 5,

- d'autre part, en eau distillé apyrogène stérile, de rinçage, d'élution ou de régénération provenant de la cuve 6 via la conduite 7,

- d'autre part encore, en solution aqueuse alcaline d'élution ou de régénération, provenant de la cuve 8 via la conduite 9,

- et finalement, en solution aqueuse d'acide chlorhydrique de régénération provenant de la cuve 10, via la conduite 11.

L'effluent de la colonne 1 est envoyé via la conduite 12 et la pompe 13 dans la cuve tampon 14, munie d'un agitateur 15, d'où il est refoulé via la conduite 16 et la pompe 17 dans un appareil d'ultrafiltration 18 ; l'ultrafiltrat est évacué par la conduite 19 dans la cuve tampon 20 munie d'un agitateur 21, et de là est envoyé à un poste de filtration stérilisante 22 puis à un poste de conditionnement ou de séchage 23.

La portion de l'effluent de la colonne 1, qui n'est ps retenue, est rejetée par la conduite d'évacuation 24.

La mise en oeuvre du procédé selon l'invention dans l'installation décrite ci-dessus se fait de manière générale comme suit :

l'hydrolysat enzymatique de protéines ultrafiltré, provenant de la cuve 3, est chargé sur la colonne de résine échangeuse de cation sous forme H ; des mesures de contrôle de l'indice de réfraction et du pH sont pratiquées sur l'effluent de la colonne ; cet effluent est rejeté par la conduite 24.

Après chargement de l'hydrolysat ultrafiltré sur la colonne, on lave la résine échangeuse par de l'eau distillée, apyrogène, stérile, provenant de la cuve 6 ; des mesures d'indice de réfraction et de pH sont pratiquées sur l'effluent de la colonne ; cet effluent est également rejeté par la conduite 24.

On procède alors à l'élution de la colonne de résine par une solution aqueuse d'alcali (soude ou potasse) provenant de la cuve 8. On suit, attentivement, l'indice de réfraction et le pH de l'effluent de la colonne ; on rejette cet effluent par la conduite 24 tant que l'indice de réfraction de l'effluent reste inférieur à 5 ; dès que l'indice de réfraction de l'effluent a atteint la valeur 5, on ferme la vanne 25 montée sur la conduite 24 et on ouvre la vanne 26 pour envoyer l'effluent dans la cuve tampon 14 via la conduite 12 et la pompe 13 ; dès que le pH de l'effluent a atteint la valeur 8, on ferme la vanne 26 et on rouvre la vanne 25 pour rejeter l'effluent par la conduite 24.

La fraction de l'effluent de la colonne qui sort de celle-ci entre un indice de réfraction de 5 et un pH de 8, qui est la fraction enrichie en di-et tri-peptides, et qui est recueillie dans la cuve 14 est soumise à une ultrafiltration dans l'ultrafiltre 18, recueillie dans la cuve tampon 20, soumise à une filtration stérilisante au poste 22, puis conditionnée ou séchée dans le poste 23.

Eventuellement une étape de concentration ou de dilution peut être prévue avant ou après l'ultrafiltration faite en 18.

La résine épuisée est régénéré :

- par traitement par la solution aqueuse alcaline provenant de la cuve 8,

- lavage par de l'eau, de préférence stérile et apyrogène,

- acidification par la solution aqueuse d'acide chlorhydrique provenant de la cuve 10,

- rinçage par de l'eau, de préférence stérile et apyrogène.

On décrit l'invention en référence aux exemples non limitatifs qui suivent, donnés uniquement à titre d'illustration.

## EXEMPLE 1

On part d'un hydrolysat enzymatique de caséine, obtenu par hydrolyse de la caséine lactique alimentaire en milieu alcalin par le Neutrase®, l'Alcalase® et la Pem ® (cas trois préparations enzymatiques, dont les deux premières sont des protéases bactériennes et la troisième un mélange d'enzymes pancréatiques, sont commercialisées par la firme NOVO INDUSTRIE ENZYMES S.A.). L'hydrolyse est suivie d'une ultrafiltration. L'hydrolysat ainsi obtenu est caractérisé par un degré d'hydrolyse (D.H.) supérieur à 33 % et contient moins de 8 % d'aminoacides libres. Le calcuil montre que pour un tel hydrolysat, la teneur en di-et tri-peptides est voisine de 50 % en poids.

4 volumes (par rapport au volume de la colonne 3) de cet hydrolysat à 11 g/litre d'azote sont déposés sur la résine échangeuse de cations sous forme H , en l'occurrence la résine Duolite® type XE 521-251 (commercialisée par la firme DUOLITE INTERNATIONAL) contenue dans la colonne 3.

L'effluent de la colonne est suivi en indice de réfraction et en pH.

Après chargement de l'hydrolysat, la colonne est d'abord lavée par 3 volumes d'eau distillée apyrogène, stérile, puis éluée par une solution aqueuse d'hyroxyde de sodium à 35 g/l.

On récupère la fraction de l'éluat enrichie en di-et tri-peptides qui sort de la colonne, dès que l'indice de réfraction de l'effluent a atteint la valeur 5 et on arrête la récupération dès que le pH de cette fraction a atteint la valeur 8.

Cette fraction est soumise à une ultrafiltration puis à une filtration stérilisante.

Le rendement en azote est compris entre 40 et 60%, le rendement en di-et tri-peptides est de 75%.

Les opérations sont conduites stérilement dans l'eau apyrogène, à une température comprise entre 60 et 80°C, et à un débit de 2 volumes/heure (en m3/heure).

La composition et les caractéristiques de la fraction de l'éluat enrichie en di-et tri-peptides, qui constitue le produit désiré selon l'invention, sont mentionnées dans le tableau I ; celles de l'hydrolysat enzymatique ultrafiltré de départ y sont également reportées.

## TABLEAU I

Composition et caractéristiques du produit selon l'invention, et celles de l'hydrolysat de départ

(Hydrolysat de caséine)

| | Hydrolysat ultra-filtré | Fraction de l'éluat enrichie en di- et tri-peptides |
|---|---|---|
| Azote total | 11 g/1 | 12,5 g/1 |
| Osmolarité | 490 mOs/kg | 250 mOs/kg |
| pH | 7,6 | 7 |
| Sodium | 80 meq/1 | $<$5 meq/1 |
| Potassium | 35 meq/1 | $<$5 meq/1 |
| Calcium | 10 meq/1 | 0 |
| Ammoniaque | 25 meq/1 | $<$10 meq/1 |
| Chlore | 25 meq/1 | $<$10 meq/1 |
| Phosphore total | 0,8 g/1 | 50 mg/1 |
| Stérilité | non stérile | stérile |
| Pyrogénicité | pyrogène | apyrogène |
| Répartition des peptides | | |
| Aminoacides libres | 7 % en poids | 3 % |
| Dipeptides | 25 % | 38 % |
| Tripeptides | 25 % | 38 % |
| Tétrapeptides | 25 % | 19,5 % |
| Teneur en di-, tri-peptides | 50 % | 76 % |
| Teneur en di-,tri-tétra- peptides | 75 % | 95,5 % |
| Oligopeptides (supérieurs aux tétra-peptides) | 18 % | 1,5 % |

Le teneur en acides aminés, sous forme libre ou sous forme peptidique , de la fraction de l'éluat enrichie en di-et tri-peptides est donnée dans le tableau II.

## TABLEAU II

Teneur en acides aminés de la fraction enrichie
en di- et tri- peptides

| Acides aminés | % en poids, sous forme peptidique | % en poids, sous forme libre |
|---|---|---|
| Acide Aspartique + Asparagine | 96,8 | 3,2 |
| Thréonine | 96,1 | 3,9 |
| Sérine | 96 | 4 |
| Acide Glutamique + Glutamine | 97,7 | 2,3 |
| Proline | 100 | 0 |
| Glycine | 90,4 | 9,6 |
| Alanine | 91,8 | 8,2 |
| Valine | 97,1 | 2,9 |
| 1/2 Cystine | 100 | 0 |
| Méthionine | 95,5 | 4,5 |
| Isoleucine | 100 | 0 |
| Leucine | 85,2 | 14,8 |
| Tyrosine | 91,8 | 8,2 |
| Phénylalanine | 98,5 | 1,5 |
| Lysine | 94,6 | 5,4 |
| Histidine | 98,3 | 1,7 |
| Tryptophane | 100 | 0 |
| Arginine | 80 | 20 |

Le bilan peptidique global établi par rapport à l'hydrolysat ultrafiltré est donné dans le tableau III.

## TABLEAU III

Bilan peptidique global par rapport à
l'hydrolysat ultrafiltré.

|  | Récupération | Elimination |
|---|---|---|
| Aminoacides libres | 25 % | 75 % |
| Dipeptides | 74 % | 26 % |
| Tripeptides | 78 % | 22 % |
| Tétrapeptides | 44 % | 56 % |
| Polypeptides (supérieurs aux pentapeptides) | 5 % | 95 % |

A titre d'exemple, le tracé de la courbe des indices de réfraction IR (courbe I) et le tracé de la courbe des pH (courbe II) des diverses fractions de l'effluent lors des phases suivantes :

- chargement de l'hydrolysat : phase A
- lavage par $H_2O$ : phase B
- élution au KOH, début d'élution : phase C
- élution au KOH, récupération de la fraction enrichie en di-et tri-peptides : phase D

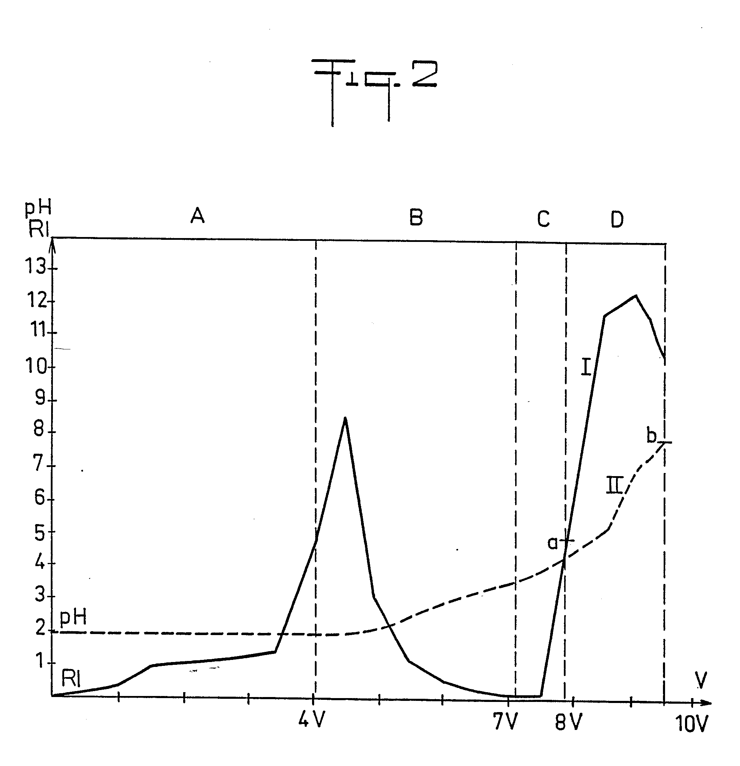

(avec début de récupération, point a, IR = 5 de la courbe I, et fin de récupération, point b, pH = 8 de la courbe II) sont représentés sur le graphique de la figure 2 donnant l'indice de réfraction et le pH en ordonnées et le volume de l'effluent en V (volume de la colonne de résine) en abscisses, pour un volume de colonne de résine échangeuse de cations sous forme acide de 200 cm3.

La fraction d'effluent obtenue lors de la phase A de chargement de l'hydrolysat a un volume de 4 V ; un indice de réfractrion augmentant de 0 à 5, un pH de 2; celle obtenue lors du lavage à l'eau a un volume de 3 V . un indice de réfraction croissant de 5 à 8,5 puis décroissant jusqu'à zéro, un pH croissant de 2 à 3,5 ; celle obtenue lors de l'élution au KOH, au début de l'élution à un volume de 0,75 V, un indice de réfraction croissant de 0 à 5, un pH croissant de 3,5 à 4,5 ; la fraction d'éluat enrichie en di-et tri-peptides, récupérée entre IR = 5 (pH = 4,5) et pH = 8, a un volume de 1,75 V.

Le tableau IV donne la composition en amin acides de la caséine matière première, de l'hydrolysat ultrafiltré et du produit fini selon l'invention.

## TABLEAU IV

Composition en aminoacides de la matière première, de l'hydrolysat ultrafiltré et du produit fini selon l'invention.

( Résidus en pour cent )

| | Matière première | Hydrolysat ultra-filtré | Produit selon l'invention |
|---|---|---|---|
| Acide aspartique et asparagine | 6,6 | 7,0 | 5,7 |
| Thréonine | 5,6 | 5,7 | 4,4 |
| Sérine | 7,2 | 6,8 | 4,4 |
| Acide glutamique et glutamine | 20,8 | 19,4 | 17,0 |
| Proline | 12,3 | 12,0 | 13,0 |
| Glycine | 3,0 | 2,9 | 3,3 |
| Alanine | 4,0 | 4,2 | 5,0 |
| Cystéine | 0,5 | 2,9 | 4,0 |
| Valine | 6,9 | 6,6 | 6,5 |
| Méthionine | 1,3 | 1,3 | 3,0 |
| Isoleucine | 2,7 | 3,0 | 4,3 |
| Leucine | 8,6 | 8,3 | 10,7 |
| Tyrosine | 3,7 | 3,4 | 4,8 |
| Phénylalanine | 4,1 | 3,9 | 4,6 |
| Lysine | 7,4 | 7,3 | 7,8 |
| Histidine | 2,4 | 2,4 | 2,7 |
| Tryptophane | non déterminé | - | - |
| Arginine | 2,9 | 2,9 | 2,4 |

# 0 274 939

EXEMPLE 2

On part d'un hydrolysat enzymatique d'ovalbumine dénaturé obtenu par l'hydrolyse du blanc d'oeuf par l'Alcalase®, par la Neutrase® et la Pem®. Ces trois préparations enzymatiques sont commercialisées par la firme NOVO INDUSTRIE ENZYMES S.A. L'hydrolysat ainsi obtenu est caractérisé par un degré d'hydrolyse (D.H.) supérieur à 30 % et contient moins de 10 % d'aminoacides libres. Le calcul montre que pour un tel hydrolysat, la teneur en di-et tri-peptides est voisine de 45 % en poids. L'hydrolyse est suivie d'une ultrafiltration.

4 volumes (par rapport au volume de la colonne) de cet hydrolysat dilué à 9,5g/litre d'azote sont déposés sur là résine échangeuse de cations sous forme H , en l'occurence la résine Amberlite® type 200C (commercialisée par la firme ROHM et HAAS) contenue dans la colonne 3.

L'effluent de cette colonne est suivi en indice de réfraction et en pH.

Après chargement de la colonne en hydrolysat, la colonne est d'abord lavée par 3 columes d'eau, puis éluée par une solution aqueuse normale d'hydroxyde de potassium.

On recueille la fraction de l'éluat qui sort de la colonne dès que l'indice de réfraction de cet éluat a atteint la valeur 5 et on arrête la récupération dès que le pH de cette fraction a atteint la valeur 8.

Cette fraction récupérée est soumise à une ultrafiltration puis à une filtration stérilisante.

Le rendement en azote est de 40 % et le rendement en di-et tri-peptides est d'environ 55 %.

Les opérations sont conduites stérilement, de préférence dans l'eau apyrogène, à une température comprise entre 60 et 80°C, à un débit de 1,5 à 2,5 volumes par heure.

La composition et les caractéristiques de la fraction de l'éluat enrichie en di-et tri-peptides qui constitue le produit désiré selon l'invention sont mentionnées dans le tableau V ainsi que celles de l'hydrolysat ultrafiltré de départ.

Le tableau VI donne pour chaque acide aminé la teneur sous forme peptidique et sous forme libre.

Le tableau VII présente le bilan de la séparation-purification sur colonne échangeuse de cations.

Le tableau VIII donne la composition en aminoacides du blanc d'oeuf matière première, de l'hydrolysat ultrafiltré et du produit fini selon l'invention.

12

## TABLEAU V

Composition et caractéristiques du produit selon
l'invention, et celles de l'hydrolysat de départ

(Hydrolysat d'ovalbumine)

|  | Hydrolysat ultra-filtré sous forme concentrée | Fraction de l'éluat enrichie en di- et tri- peptides |
|---|---|---|
| Azote total | 15 g/l | 13 g/l |
| Osmolarité | 900 mOs/kg | 315 mOs/kg |
| pH | 7,0 | 5,5 |
| Sodium | 150 meq/l | 1,7 meq/l |
| Potassium | 40 meq/l | 0,6 meq/l |
| Calcium | 550 mg/l | 32 mg/l |
| Chlore | 20 meq/l | 1,2 meq/l |
| Phosphore total | 18 mg/l | 3 mg/l |
| Stérilité | non stérile | stérile |
| Pyrogénicité | pyrogène | apyrogène |
| Répartition des protides |  |  |
| Aminoacides libres | 11 % | 9 % |
| Dipeptides | 20,5 % | 30,0 % |
| Tripeptides | 20,5 % | 30,0 % |
| Tétrapeptides | 20,0 % | 13,0 % |
| Teneur en di-, tri-peptides | 41 % | 60,0 % |
| Teneur en di-, tri-tétra- peptides | 61 % | 73,0 % |
| Oligopeptides (supérieurs aux tétra-peptides) | 28 % | 18,0 % |

TABLEAU VI

Teneur en acides aminés de la fraction enrichie
en di- et tri- peptides

(Hydrolysat d'ovalbumine)

| Acides aminés | % en poids, sous forme peptidique | % en poids, sous forme libre |
|---|---|---|
| Acide Aspartique + Asparagine | 91,3 % | 8,7 % |
| Thréonine | 93,7 % | 6,3 % |
| Sérine | 79,0 % | 21,0 % |
| Acide Glutamique + Glutamine | 90,8 % | 9,2 % |
| Proline | 100 % | 0 % |
| Glycine | 92,1 % | 7,9 % |
| Alanine | 90,0 % | 10,0 % |
| Valine | 97,1 % | 2,9 % |
| 1/2 Cystine | ND | ND |
| Méthionine | 91,6 % | 8,4 % |
| Isoleucine | 100 % | 0 % |
| Leucine | 87,0 % | 13,0 % |
| Tyrosine | 89,0 % | 11,0 % |
| Phénylalanine | 87,0 % | 13,0 % |
| Lysine | 90,0 % | 10,0 % |
| Histidine | 96,0 % | 4,0 % |
| Tryptophane | ND | ND |
| Arginine | 95,0 % | 5,0 % |

ND : non déterminé.

TABLEAU VII

Bilan peptidique global par rapport à
l'hydrolysat ultrafiltré

( Ovalbumine )

|  | Récupération | Elimination |
|---|---|---|
| Aminoacides libres | 23 % | 77 % |
| Dipeptides | 42 % | 58 % |
| Tripeptides | 42 % | 58 % |
| Tétrapeptides | 19 % | - 81 % |
| Polypeptides (supé-rieurs aux pentapeptides) | 18 % | 82 % |

# 0 274 939

## TABLEAU VIII

Composition en aminoacides de la matière première, de l'hydrolysat ultrafiltré et du produit fini selon l'invention.

( Résidus en pour cent )

| | Matière première | Hydrolysat ultra-filtré | Produit selon l'invention |
|---|---|---|---|
| Acide aspartique et asparagine | 10,1 | 11,2 | 10,0 |
| Thréonine | 5,4 | 5,6 | 4,9 |
| Sérine | 7,6 | 8,7 | 8,0 |
| Acide glutamique et glutamine | 12,1 | 12,6 | 10,3 |
| Proline | 3,7 | 3,4 | 3,5 |
| Glycine | 6,9 | 6,6 | 7,5 |
| Alanine | 9,1 | 9,7 | 10,7 |
| Cystéine | 1,9 | 2,0 | ND |
| Valine | 7,1 | 7,5 | 6,7 |
| Méthionine | 4,0 | 3,3 | 3,8 |
| Isoleucine | 4,2 | 4,3 | 4,3 |
| Leucine | 8,1 | 7,7 | 8,0 |
| Tyrosine | 2,8 | 2,1 | 2,6 |
| Phénylalanine | 4,2 | 3,5 | 4,0 |
| Lysine | 5,9 | 6,0 | 8,5 |
| Histidine | 1,8 | 1,8 | 2,6 |
| Tryptophane | 0,9 | 0,7 | ND |
| Arginine | 4,2 | 4,0 | 4,6 |

ND = non déterminé

EXEMPLE 3

Cet exemple est relatif à la présentation pharmaceutique d'un aliment diététique pour alimentation entérale.

L'hydrolysat enrichi en di-et tri-peptides selon l'invention est préparé selon l'exemple 1 ou 2, puis il est dilué de façon à obtenir 30-60 g de protides/litre.

A la solution peptidique ainsi obtenue sont ajoutés les hydrates de carbone sous forme de maltodextrines, des lipides sous forme d'huiles végétales, des vitamines, des sels minéraux, des oligoéléments ainsi que plusieurs additifs technologiques nécessaires à la tenue en émulsion du produit fini. L'aliment entéral ainsi constitué est stérilisé par la technique UHT, (UHT = Ultra Haute température), puis il est réparti sous un volume compris entre 200 et 1000ml, soit en boîtes métalliques, soit en emballages en carton Tétrabrik®, soit encore dans un autre conditionnement.

A titre d'exemple, une formule à 4180 kJ/l (soit 1000 kcal/l) ayant une osmolarité comprise entre 300 et 450 mOs/kg présente la composition suivante :

| | |
|---|---|
| - hydrolysat purifié et enrichi en petits peptides. Volume correspondant à la matière sèche : | 21 g |
| - maltodextrines | 60 g |
| - amidon | 10 g |
| - mélange lipidique (huile de soja, colza, onagre) | 8 g |
| - triglycérines à chaines moyennes | 8,7 g |
| - mélange vitaminique | 0,1 g |
| - sulfate de magnésium | 0,7 g |
| - chlorure de calcium | 0,2 g |
| - glycérophosphate de calcium | 0,8 g |
| - sulfate de fer (ferreux) | 0,01 g |
| - carbonate de manganèse | 0,003 g |
| - sulfate de zinc | 0,008 g |
| - sulfate de cuivre | 0,002 g |
| - iodure de potassium | 0,05 mg |
| - émulsifiant | 1,3 g |
| - antioxydant des huiles | 0,003 g |
| - eau distillée q.s.p. | 500 ml |

EXEMPLE 4

Cet exemple est relatif à la présentation pharmaceutique d'un soluté injectable d'apport azoté utilisable en alimentation parentérale.

L'hydrolysat enrichi en di-et tri-peptides selon l'invention est préparé selon l ' exemple 1 ou 2 et comme indiqué sur la figure 2.

17

Avant répartition dans des locaux stériles, et conformément aux bonnes pratiques de fabrication de l'industrie pharmaceutique, on ajoute 0,5-1,5 g par litre d'hydrosulfite de sodium comme agent antioxygène.

Le produit est enfin stérilisé à l'autoclave (120°C, 20 min.) en flacons de verre de 125, 250, 500 ou encore 1000 ml.

## EXEMPLE 5

Cet exemple est relatif à la présentation pharmaceutique d'un concentré peptidique utilisable pour les perfusions en veine profonde et en mélange extemporané avec des lipides injectables et du glucose injectable (alimentation parentérale totale). La fabrication est similaire à celle de l'exemple 4, mais comporte en outre une opération de concentration pour augmenter la teneur en azote protidique du produit à une valeur comprise entre 15 et 30 g d'azote/litre.

Le produit ainsi obtenu est ensuite conditionné et présenté comme le produit décrit dans l'exemple 4.

## Revendications

1. Procédé de préparation d'un mélange peptidique riche en di-et tri-peptides utilisable notamment en nutrition artificielle et en diététique, à partir d'un hydrolysat enzymatique de protéines contenant des di-, tri-peptides et des polypeptides supérieurs aux tri-peptides, ledit procédé comprenant :
- la chromatographie de cet hydrolysat sur une colonne d'une résine échangeuse de cations sous forme acide,
- le lavage de la colonne de résine échangeuse par de l'eau, de préférence stérile, apyrogène,
- l'élution de ladite colonne de résine échangeuse par une solution aqueuse d'un alcali,
- et la récupération de la fraction de l'éluat enrichie en di-et tri-peptides.

2. Procédé selon la revendication 1, caractérisé en ce que la fraction de l'éluat enrichie en di-et tri-peptides à récupérer est celle qui sort de la colonne de résine échangeuse depuis le moment où l'indice de réfraction de cet éluat a atteint la valeur 5, jusqu'au moment où le pH de cet éluat a atteint la valeur 8, moment auquel la récupération de ladite fraction est terminée.

3. Procédé selon la revendication 1 ou 2, caractérisé en ce que :
- l'on chromatographie, sur la colonne de résine échangeuse de cations sous forme H , 2 à 5 volumes d'hydrolysat enzymatique de protéines (par rapport au volume de la colonne), selon la concentrtion en protides de l'hydrolysat, la quantité totale d'hydrolysat chargée sur la colonne étant égale ou inférieure à celle de saturation de la résine échangeuse, qui correspond à 400g environ de protides par litre de résine échangeuse.
- on lave la colonne de résine échangeuse par 2 à 3 volumes d'eau,
- on élue la colonne de résine échangeuse par une solution aqueuse de soude ou de potasse à une concentration égale ou inférieure à 2 N,
- on suit l'indice de réfraction et le pH de l'éluat, et on récupère la fraction de l'éluat qui sort de la colonne de résine échangeuse depuis le moment où l'indice de réfraction de cet éluat a atteint la valeur 5, jusqu'au moment où le pH de cet éluat a atteint la valeur 8, cette fraction de l'éluat constituant le mélange peptidique riche en di-et tri-peptides.

4. Procédé selon l'une quelconque des revendications 1 à 3 précédentes, caractérisé en ce que la fraction de l'éluat enriche en di-et tri-peptides est purifiée par ultrafiltration.

5. Procédé selon la revendication 4, caractérisé en ce qu'une étape de concentration ou de dilution est prévue avant ou après l'ultrafiltration.

6. Procédé selon la revendication 4, caractérisé en ce que la fraction de l'éluat enrichie en di-et tri-peptides purifiée par ultrafiltration est soumise à une filtration stérilisante puis conditionnée sous forme d'un liquide ou bien séchée sous la forme d'une poudre.

7. Procédé selon l'une quelconque des revendications 1 à 3 précédentes, caractérisé en ce que la température est maintenue à 4-80°C, de préférence à 60-80°C.

8. Procédé selon l'une quelconque des revendications 1 à 3 précédentes, caractérisé en ce que la solution aqueuse d'acali est une solution aqueuse de soude ou une solution aqueuse de potasse de concentration inférieure ou égale à la normalité.

9. Procédé selon l'une quelconque des revendications 1 à 3, caractérisé en ce que l'hydrolysat enzymatique de protéines est un produit de digestion enzymatique du blanc d'oeuf ou de la caséine.

10. Procédé selon l'une quelconque des revendications 1 à 3, caractérisé en ce que la résine échangeuse de cations sous forme acide est une résine cationique fortement acide, à squelette polystyrénique, portant des groupements acides sulfoniques, et présentée sous forme de solide macroporeux.

11. Mélange peptidique riche en di-et tri-peptides obtenu directement par le procédé selon l'une quelconque des revendications 1 à 10.

12. Utilisation du mélange peptidique riche en di-et tri-peptides selon la revendication 11 pour la confection des compositions pour nutrition artificielle orale, entérale.

13. Utilisation du mélange peptidique riche en di-et tri-peptides selon la revendication 11 pour la confection des compositions pour nutrition artificielle parentérale.

14. Utilisation du mélange peptidique riche en di-et tri-peptides selon la revendications 11 pour la confection des compositions diététiques.

15. Installation pour la mise en oeuvre du procédé de préparation d'un mélange peptidique riche en di-et tri-peptides à partir d'un hydrolysat enzymatique de protéines selon l'une quelconque des revendications 1 à 10, caractérisée en ce qu'elle comporte une colonne 1 de résine échangeuse de cations sous forme acide, ladite colonne étant pourvue :
- d'une part à sa partie supérieure :
. d'une arrivée 4 d'hydrolysat de protéines ultrafiltré ,
. d'une arrivée 7 d'eau distillée apyrogène, stérile de rinçage, d'élution ou de régénération,
. d'une arrivée 9 de solution aqueuse alcaline d'élution ou de régénération,
. d'une arrivée 11 de solution aqueuse d'acide de régénération,
- d'autre part, à sa partie inférieure :
. d'une sortie 12 pour évacuer l'effluent de la colonne, cette sortie étant éventuellement reliée à un appareil d'ultrafiltration 18 puis à un filtre stérilisant 22 et ensuite à un poste de conditionnement et de séchage 23,
. et finalement de moyens pour le contrôle de l'indice de réfraction et du pH de l'effluent de la colonne.

Fig. 1

0 274 939

Fig. 2